# EUROPEAN PATENT APPLICATION

(11) **EP 2 246 046 A1**
(43) Date of publication of application: **03.11.2010**
(21) Application number: 10161187.9
(22) Date of filing: 27.04.2010
(51) Int. Cl.: A61K 9/20, A61K 31/5513

(54) **Orally disintegrating olanzapine tablet**

(30) Priority: 28.04.2009 TR 200903293
(71) Applicant: Sanovel Ilac Sanayi ve Ticaret A.S., 34398 Istanbul (TR)
(72) Inventor: Cifter, Ümit, 34398, Istanbul (TR); Türkyilmaz, Ali, 34398, Istanbul (TR); Yasar, Tuba M., 34398, Istanbul (TR); Dogu, Yücel, 34398, Istanbul (TR)
(74) Representative: Sevinç, Erkan

(57) **Abstract**

According to the present invention an orally disintegrating olanzapine tablet comprising magnesium stearate and sodium stearyl fumarate and one or more pharmaceutically acceptable excipient in which the total weight of magnesium stearate and sodium stearyl fumarate is about 0,1 to 5% by weight of the total tablet and wherein the tablet disintegrates within up to 90 seconds in oral cavity is provided.

## Description

### Technical Aspect

The present invention relates to an orally disintegrating olanzapine tablet comprising magnesium stearate and sodium stearyl fumarate and one or more pharmaceutically acceptable excipient in which the total weight of magnesium stearate and sodium stearyl fumarate is about 0,1 to 5% by weight of the total tablet and wherein the tablet disintegrates within up to 90 seconds in oral cavity.

### Background of the Invention

Olanzapine is a psychotropic agent that belongs to the thienobenzodiazepine class. The chemical name is 2-methyl-4-(4-methyl-1-piperazinyl)-10H-thieno[2,3-b] [1,5] benzodiazepine and the chemical structure is shown in the Formula I.

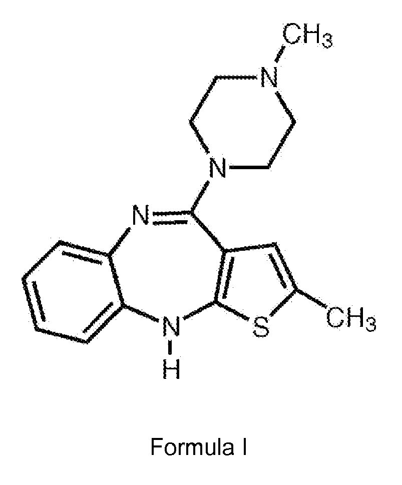

Olanzapine is marketed under the brand name Zyprexa® and Zyprexa Zydis® for the treatment of psychotic disorders such as schizophrenia, acute mania in bipolar disorder, and agitation associated with schizophrenia and bipolar disorder.

Olanzapine is a selective monoaminergic antagonist with high affinity binding to the following receptors: serotonin 5HT_{2A/2C}, 5HT₆, (Kᵢ=4, 11, and 5 nM, respectively), dopamine D₁₋₄ (Kₗ=11-31 nM), histamine H₁ (Kᵢ=7 nM), and adrenergic (alpha)₁ receptors (Kᵢ=19 nM). Olanzapine is an antagonist with moderate affinity binding for serotonin 5HT₃ (Kᵢ=57 nM) and muscarinic M₁₋₅ (Kᵢ=73, 96, 132, 32, and 48 nM, respectively). Olanzapine binds weakly to gamma-aminobutyric acid type A (GABA_{A}), benzodiazepine (BZD), and (beta) adrenergic receptors (Ki>10 µM).

PCT application WO 2003/086361 A1 (Dr. Reddy's Lab. Ltd.) 18.04.2002, discloses rapidly dispersing solid oral compositions comprising olanzapine or ondansetron. The composition is manufactured by wet granulation or direct compression.
PCT application WO 2006/074951 A2 (Krka) 14.01.2005, discloses orally disintegrating composition comprising olanzapine or donepezil together with calcium silicate and mannitol.

PCT application WO 2006/092812 A2 (Actavis Group) 02.03.2005, discloses rapidly disintegrating dosage form containing magnesium carbonate heavy as a dispersant.

The development of solid dosage forms that disintegrate quickly in the mouth without requiring water has advantage for patients who have difficulty in swallowing, such as geriatric and pediatric patients, patients with mental problems and non- compliant patients since it makes possible for the drug to be administered without the need for water.

Oral administration of the drugs is difficult in patients having concomitant vomiting or diarrhoea. The orally disintegrating dosage form is one of the advantageous methods to deliver the drugs to such patients. By administering the orally disintegrating dosage forms, faster absorption of the drug occurs through buccal mucosa and it may reduce the first pass metabolism leading to better efficacy of the drug. This dosage form enhances the clinical effects of some drugs by leading to an increase in bioavailability and a reduction in side effects because of avoidance of first-pass liver metabolism.

It is difficult to develop orally disintegrating compositions because of several different reasons. First of all, the time in which dosage form must disintegrate in the oral cavity with the existence of saliva has to be much shorter than it should be in stomach. So those compositions should be very porous and should not be very hard. These porous compositions tend to be very sensitive to humidity. As a consequence, they may have some stability problems. Additionally, orally disintegrating compositions need to take precautions in the preparation, packaging, handling and storing of the finished dosage forms.

### Description of the invention

According to the present invention an orally disintegrating olanzapine tablet comprising magnesium stearate and sodium stearyl fumarate and one or more pharmaceutically acceptable excipient in which the total weight of magnesium stearate and sodium stearyl fumarate is about 0,1 to 5% by weight of the total tablet and wherein the tablet disintegrates within up to 90 seconds in oral cavity is provided.

Olanzapine is reported as practically insoluble in water, which cause it to exhibit a low dissolution rate in aqueous media such as gastrointestinal fluids, which can result in low bioavailability after oral ingestion. Olanzapine is a hygroscopic drug and sensitive to heat and moisture.

It is known that magnesium stearate has some disadvantages despite being a good lubricant and because of this it is used in small quantities during drug manufacturing process. Magnesium stearate is practically insoluble in water and because of this hydrophobic characteristic it may retard the dissolution of a drug from a solid dosage form such as tablet or capsule. Tablet and especially capsule dissolution is sensitive to both the amount of magnesium stearate in the formulation and the blending time. Blending time should be limited. Long blending times can result in the formulation of hydrophobic powder beds that do not disperse easily and overblending can cause compaction problems. Tablet dissolution rate and crushing strength decreased as the time of blending increased; and magnesium stearate may also increase tablet friability. Blending times with magnesium stearate should therefore be carefully controlled. (Handbook of Pharmaceutical Excipients, fifth edition, Rowe, Raymond C., Sheskey, Paul J., Owen, Sian C., pages 430-432*).*

Sodium stearyl fumarate is extremely effective lubricant and less hydrophobic than magnesium stearate and has a less retardant effect on tablet dissolution than magnesium stearate. Sodium stearyl fumarate also doesn't have the over blending problems seen with magnesium steare. (Handbook of Pharmaceutical Excipients, fifth edition, Rowe, Raymond C., Sheskey, Paul J., Owen, Sian C., pages 705-707*).*

We have surprisingly found that, using magnesium stearate and sodium stearyl fumarate at an appropriate rate, which is about 0,1 to 5% by weight of total tablet, prevents hygroscopicity of composition during manufacturing process and sticking to punch faces, provides enhanced powder flow by reducing interparticle friction and forms orally disintegrating tablet which has better physical properties during stability. This rate is preferably about 0,5 to 4%, more preferably 1 to 4%.

It has unexpectedly found that this orally disintegrating olanzapine tablet that having a weight ratio of magnesium stearate to sodium stearyl fumarate in the range of between 1:10 to 10:1 (w/w), preferably between 1:5 to 5:1 (w/w) has positive effect on the disintegration time.

The orally disintegrating compositions of the present invention may be manufactured by conventional technology well known to those skilled in the art such as wet granulation, direct compression, dry granulation and the like. The orally disintegrating compositions of the present invention may also be manufactured by other technologies such as zydis, orasolv, durasolv, wowtab and the like.

Wet granulation technique results in cores of a high hardness which make it difficult to obtain fast dissolving and fast disintegrating tablets. Wet granulation leads to coarse dispersions in the oral cavity resulting in a poor patient compliance. The use of solvents and the additional drying step make this technique expensive.

Direct compression is a commonly used tablet manufacturing process to produce orally disintegrating tablets. Because it uses existing high-speed tablet press equipment and common excipients, it is often preferred over other manufacturing processes for orally disintegrating tablets. A direct-compression formulation has better physical properties relative to other methods that may eliminate the need for special packaging such as blister packages.

The advantages of direct compression include uniformity of blend, few manufacturing steps involved, (i.e. the overall process involves weighing of powders, blending and compression, hence less cost), elimination of heat and moisture, and physical stability.

Since olanzapine is hygroscopic and sensitive to heat and moisture, it is desirable to manufacture the orally disintegrating olanzapine tablet by direct compression to prevent the negative effects of moisture. Using direct compression technique together with adequate excipients, we avoid the product to gather moisture, and in this way we achieved a stable composition throughout the shelf life.

The orally disintegrating olanzapine tablet which is manufactured by direct compression, disintegrates within up to 90 seconds, preferably 60 seconds, the more preferably 25 seconds in oral cavity.

The hardness of the tablet has effect on the disintegration time. Therefore it is desirable to ensure an orally disintegrating tablet which is soft enough to reach the desirable disintegration time at the desired time period and hard enough to overcome the negative effects of blistering process. These conditions are surprisingly provided in a range from 5 to 130 N, particularly it is about 20 to 70 N.

A preferred process for manufacturing the orally disintegrating olanzapine tablet comprises the following steps:
a. mixing olanzapine or a pharmaceutically acceptable salt or polymorph thereof with other excipients;
b. blending the mixture with magnesium stearate and sodium stearyl fumarate;
c. compressing the blended mixture by direct compression to form tablets at a pressure of 5 N to 130 N.

In one embodiment, the amount of olanzapine or a pharmaceutically acceptable salt or polymorph thereof is present in about 1 to 95% by weight of total tablet, preferably about 1 to 50%, the more preferably about 5 to 30%.

In a further aspect, the present invention relates to the orally disintegrating olanzapine tablet comprising olanzapine or a pharmaceutically acceptable salt or polymorph thereof, is in an amount of 1 to 50mg.

The compositions of the invention comprise one or more excipients. Such excipients include super disintegrants, diluents, binders, sweeteners, glidants and lubricants.

Suitable super disintegrants may include but not limited to starch, sodium starch glycollate, crospovidone, low-substituted hydroxypropylcellulose, croscarmellose sodium and the like and mixtures thereof, preferably low-substituted hydroxypropylcellulose and crospovidone.

In preferred embodiments, the amount of low-substituted hydroxypropylcellulose is present in about 1 to 50% by weight of total tablet, particularly about 1 to 20% and the amount of crospovidone is present in about 0,2 to 20% by weight of total tablet, particularly about 1 to 15%.

Suitable diluents may include but not limited to lactose, microcrystalline cellulose, spray-dried mannitol, starch, sodium carbonate, sodium bicarbonate and the like and mixtures thereof; preferably microcrystalline cellulose and spray-dried mannitol.

In other preferred embodiments of present invention, the amount of microcrystalline cellulose is present in about 2 to 75% by weight of total tablet, preferably about 4 to 60% and the amount of spray-dried mannitol is present in about 2 to 95% by weight of total tablet, preferably about 5 to 60%.

Spray-dried mannitol provides a highly compactible, smooth mouthfeel, nonhygroscopic, free-flowing composition.

Suitable binders may include but not limited to mixture of microcrystalline cellulose and guar gum(Avicel® CE 15), polyethylene glycol, cellulose derivatives such as hydroxypropyl methyl cellulose, carboxy methyl cellulose, methyl cellulose, gelatin, polyvinylalcohol, carrageenan, guar gum, xanthan gum and the like and mixtures thereof; preferably mixture of microcrystalline cellulose and guar gum(Avicel® CE 15).

In other preferred embodiments of present invention, the amount of mixture of microcrystalline cellulose and guar gum (Avicel® CE 15) is present in about 2 to 50% by weight of total tablet, preferably about 2 to 30%.

Suitable sweeteners may include but not limited to aspartame, sucralose, saccharin, glucose, fructose, sugar alcohols e.g. mannitol, sorbitol, xylitol, erythritol and the like and mixtures thereof, preferably sucralose.

In other preferred embodiments of present invention, the amount of sucralose is present in about 0,01 to 1% by weight of total tablet, preferably about 0,2 to 1%.

Suitable glidants may include but not limited to colloidal silicon dioxide, talc, aluminum silicate and the like and mixtures thereof, preferably colloidal silicon dioxide.

In other preferred embodiments of present invention, the amount of colloidal silicon dioxide is present in about 0,05 to 4% by weight of total tablet, preferably about 0,1 to 2%.

The orally disintegrating olanzapine tablet according to present invention is comprising (a) about 1 to 95% by weight of olanzapine or pharmaceutically acceptable salt or polymorph thereof, (b) about 2 to 95% by weight of spray-dried mannitol, (c) about 1 to 50% by weight of low-substituted hydroxypropylcellulose, (d) about 0,2 to 20% by weight of crospovidone, (e) about 2 to 50% by weight of mixture of microcrystalline cellulose and guar gum (Avicel® CE 15), (f) about 2 to 75% by weight of microcrystalline cellulose, (g) about 0,01 to 1% by weight of sucralose, (h) about 0,05 to 4% by weight of colloidal silicon dioxide (i) about 0,1 to 5% by weight of sodium stearyl fumarate, (j) about 0,1 to 5% by weight of magnesium stearate, in order to be orally disintegrating and be hard enough for transporting and commercializing.

The present invention provides an orally disintegrating olanzapine tablet comprising magnesium stearate and sodium stearyl fumarate and one or more pharmaceutically acceptable excipient which is stable throughout the shelflife and which has high bioavailability.

The invention is further defined by reference to the following example. Although the example is not intended to limit the scope of the present invention, it should be considered in the light of the description detailed above.

It will be apparent to those skilled in the art that many modifications, both to materials and methods, may be practiced without departing from the scope of the invention.

### Example 1:

### 5 mg orally disintegrating olanzapine tablet

| **Active ingredient** | **mg** |
|---|---|
| Olanzapine | 5,00 |

| **Excipients** | |
|---|---|
| Microcrystalline cellulose PH 112 | 24.29 |
| Spray-dried mannitol | 10.00 |
| Low-substituted hydroxypropylcellulose LH-11 | 2.00 |
| Crospovidone CI | 6.00 |
| Mixture of microcrystalline cellulose and guar gum (Avicel® CE 15) | 2.50 |
| Sucralose | 0.40 |
| Colloidal silicon dioxide | 0.25 |
| Sodium stearyl fumarate | 0.78 |
| Magnesium stearate | 0.78 |
| **Total** | **52 mg** |

### Example 2:

### 20 mg orally disintegrating olanzapine tablet

| **Active ingredient** | **mg** |
|---|---|
| Olanzapine | 20.00 |
| **Excipients** | |
| Microcrystalline cellulose PH 112 | 38.58 |
| Spray-dried mannitol | 20.00 |
| Low-substituted hydroxypropylcellulose LH-11 | 4.00 |
| Crospovidone CI | 12.00 |
| Mixture of microcrystalline cellulose and guar gum (Avicel® CE 15) | 5.00 |
| Sucralose | 0.80 |
| Colloidal silicon dioxide | 0.50 |
| Sodium stearyl fumarate | 1.56 |
| Magnesium stearate | 1.56 |
| **Total** | **104 mg** |

### Example 3:

### 20 mg orally disintegrating olanzapine tablet

| **Active ingredient** | **mg** |
|---|---|
| Olanzapine | 20.00 |
| **Excipients** | |
| Microcrystalline cellulose PH 112 | 25,45 |
| Spray-dried mannitol | 20.00 |
| Low-substituted hydroxypropylcellulose LH-11 | 4.00 |
| Crospovidone CI | 12.00 |
| Mixture of microcrystalline cellulose and guar gum (Avicel® CE 15) | 5.00 |
| Sucralose | 0.80 |
| Colloidal silicon dioxide | 0.50 |
| Sodium stearyl fumarate | 1.25 |
| Magnesium stearate | 15,00 |
| **Total** | **104 mg** |

**Table 1:Hardness, Friability and Disintegration Time Results**

| | **Mg stearate/Sodium** **stearyl fumarate** **ratio** | **Hardness** | **Friability** | **Disintegration Time** |
|---|---|---|---|---|
| **Example 1** | 1:1 | 21N | <%0,1 | 15 sec. |
| **Example 2** | 1:1 | 22N | <%0,1 | 17 sec. |
| **Example 3** | 12:1 | 12N | %0,6 | 50 sec. |

The pharmaceutical composition of example 3 which is used for comparison of hardness, friability and dissolution tests, is comprising the same amounts of example 2, but including magnesium stearate and sodium stearyl fumarate in a different range. As it is shown in Table 1 friability and disintegration time results of Ex 1 and 2 are better than these results of example 3. These results show that the selected ratio of magnesium stearate to sodium stearyl fumarate has unexpected effects over the friability and disintegration time.

Therefore, further aspects of the present invention concern the use of pharmaceutical compositions for the treatment of psychotic disorders such as schizophrenia, acute mania in bipolar disorder, and agitation associated with schizophrenia and bipolar disorder in a warm- blooded animal.

## Claims

1. The orally disintegrating olanzapine tablet comprising magnesium stearate and sodium stearyl fumarate and one or more pharmaceutically acceptable excipient in which the total weight of magnesium stearate and sodium stearyl fumarate is about 0,1 to 5% by weight of the total tablet and wherein the tablet disintegrates within up to 90 seconds in oral cavity.

2. The orally disintegrating olanzapine tablet according to claim 1, wherein the weight ratio of magnesium stearate to sodium stearyl fumarate is in the range of between 1:10 to 10:1 (w/w)

3. The orally disintegrating olanzapine tablet according to claim 2, wherein the weight ratio of magnesium stearate to sodium stearyl fumarate is in the range of between 1:5 to 5:1 (w/w)

4. A process for manufacturing the orally disintegrating olanzapine tablet according to claim 1 and 3 is direct compression.

5. A process for manufacturing the orally disintegrating olanzapine tablet according to claims 1-4, comprising
a. mixing olanzapine, or a pharmaceutically acceptable salt or polymorph thereof with other excipients;
b. blending the mixture with magnesium stearate and sodium stearyl fumarate;
c. compressing the blended mixture by direct compression to form tablets at a pressure of 5 N to 130 N.

6. The orally disintegrating olanzapine tablet according to claim 1, wherein one or more pharmaceutically acceptable excipients are selected from the group comprising super disintegrants, diluents, binders, sweeteners, glidants and lubricants.

7. The orally disintegrating olanzapine tablet according to claim 6 wherein the super disintegrants are low-substituted hydroxypropylcellulose and crospovidone.

8. The orally disintegrating olanzapine tablet according to claim 6, wherein the diluent is microcrystalline cellulose and spray dried mannitol.

9. The orally disintegrating olanzapine tablet according to claim 6, wherein the binder is mixture of microcrystalline cellulose and guar gum.

10. The orally disintegrating olanzapine tablet according to claim 6, wherein the sweetener is sucralose.

11. The orally disintegrating olanzapine tablet according to claim 6, wherein the glidant is colloidal silicon dioxide.

12. The orally disintegrating olanzapine tablet according to any preceding claim comprising (a) about 1 to 95% by weight of olanzapine or pharmaceutically acceptable salt or polymorph thereof, (b) about 2 to 95% by weight of spray-dried mannitol, (c) about 1 to 50% by weight of low-substituted hydroxypropylcellulose, (d) about 0,2 to 20% by weight of crospovidone, (e) about 2 to 50% by weight of mixture of microcrystalline cellulose and guar gum, (f) about 2 to 75% by weight of microcrystalline cellulose, (g) about 0,01 to 1 %by weight of sucralose, (h) about 0,05 to 4% by weight of colloidal silicon dioxide (i) about 0,1 to 5% by weight of sodium stearyl fumarate, (j) about 0,1 to 5% by weight of magnesium stearate.
